# EUROPEAN PATENT APPLICATION

(11) **EP 3 252 036 A1**
(43) Date of publication of application: **06.12.2017**
(21) Application number: 16743765.6
(22) Date of filing: 01.02.2016
(51) Int. Cl.: C07C 317/32, C07C 317/14

(54) **COMPOUND COMPRISING AROMATIC RING AND POLYMER ELECTROLYTE MEMBRANE USING SAME**

(30) Priority: 30.01.2015 KR 20150014693
(71) Applicant: LG Chem, Ltd., Seoul 07336 (KR)
(72) Inventor: JANG, Yong Jin, Daejeon 34122 (KR); HAN, Joong Jin, Daejeon 34122 (KR); KIM, Youngjea, Daejeon 34122 (KR); KANG, Esder, Daejeon 34122 (KR); JUNG, Sehee, Daejeon 34122 (KR); RYU, Hyun Woog, Daejeon 34122 (KR)
(74) Representative: Hoffmann Eitle
(86) International application number: PCT/KR2016/001091
(87) International publication number: WO 2016/122287

(57) **Abstract**

The present specification relates to a compound including an aromatic ring, a polymer electrolyte membrane including the same, a membrane-electrode assembly including the polymer electrolyte membrane, a fuel cell including the membrane-electrode assembly, and a redox flow battery including the polymer electrolyte membrane.

## Description

### [Technical Field]

The present specification claims priority to and the benefits of Korean Patent Application No. 10-2015-0014693, filed with the Korean Intellectual Property Office on January 30, 2015, the entire contents of which are incorporated herein by reference.

The present specification relates to a compound including an aromatic ring and a polymer electrolyte membrane using the same.

### [Background Art]

A fuel cell is an energy conversion device directly converting chemical energy of fuel into electric energy. In other words, a fuel cell employs a power generation method utilizing a fuel gas and an oxidizer, and using electrons generated during the oxidation and reduction reactions thereof to produce power. A membrane-electrode assembly (MEA) of a fuel cell is a part where an electrochemical reaction of hydrogen and oxygen occurs, and is formed with a cathode, an anode and an electrolyte membrane, that is, an ion conductive electrolyte membrane.

A redox flow battery (oxidation-reduction flow battery) is a system charged and discharged by active materials included in a liquid electrolyte being oxidized and reduced, and is an electrochemical storage device directly storing chemical energy of the active materials as electric energy. A unit cell of the redox flow battery includes an electrode, an electrolyte and an ion-exchange membrane (electrolyte membrane).

Due to their high energy efficiency and environmental friendly properties of low contaminant emissions, fuel cells and redox flow batteries have been researched and developed as a next generation energy source.

A core constituent in a fuel cell and a redox flow battery is a polymer electrolyte membrane capable of cation exchange, and properties of 1) excellent proton conductivity, 2) preventing electrolyte cross over, 3) high chemical resistance, 4) strengthening mechanical properties and/or 4) low swelling ratio are favorably required. The polymer electrolyte membrane is divided into fluorine-based, partial fluorine-based, hydrocarbon-based and the like, and the hydrocarbon-based polymer electrolyte membrane has an advantage of low costs compared to the fluorine-based electrolyte membrane.

As materials of the hydrocarbon-based polymer electrolyte membrane, monomers having a structure in which a cation transfer functional group is directly attached to the benzene ring of the polymer main chain or is away from the polymer main chain through a carbonyl functional group have been generally used, however, studies for solving problems of reactivity and durability deterioration have been continuously required.

### [Disclosure]

### [Technical Problem]

The present specification is directed to providing a compound including an aromatic ring and a polymer electrolyte membrane using the same.

### [Technical Solution]

One embodiment of the present specification provides a compound represented by the following Chemical Formula 1: 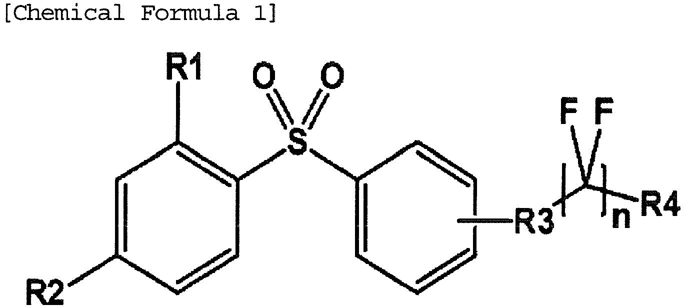
In Chemical Formula 1,
R1 and R2 are the same as or different from each other, and each independently a halogen group or a hydroxyl group,
R3 is S, O, CF₂, NH or SO₂,
R4 is -SO₃H, -SO₃⁻M⁺, -COOH, -COO⁻M⁺, -OCF₂CF₂SO₃H or - OCF₂CF₂ SO₃⁻M⁺,
M is a group 1 element, and
n is an integer of 2 to 20.

One embodiment of the present specification provides a polymer including a monomer derived from the compound of Chemical Formula 1.

One embodiment of the present specification provides a polymer electrolyte membrane including a polymer including a monomer derived from the compound of Chemical Formula 1.

In addition, one embodiment of the present specification provides a membrane-electrode assembly including an anode; a cathode; and the polymer electrolyte membrane described above provided between the anode and the cathode.

In addition, one embodiment of the present specification provides a polymer electrolyte-type fuel cell including two or more of the membrane-electrode assemblies described above; a stack including a bipolar plate provided between the membrane-electrode assemblies; a fuel supplying unit supplying fuel to the stack; and an oxidizer supplying unit supplying an oxidizer to the stack.

One embodiment of the present specification also provides a redox flow battery including a positive electrode cell including a positive electrode and a positive electrode liquid electrolyte; a negative electrode cell including a negative electrode and a negative electrode liquid electrolyte; and the polymer electrolyte membrane described above provided between the positive electrode cell and the negative electrode cell.

### [Advantageous Effects]

A monomer derived from a compound according to one embodiment of the present specification provides high reactivity in a polymerization reaction process.

A polymer electrolyte membrane prepared using a polymer including a monomer derived from a compound according to one embodiment of the present specification has excellent ion conductivity.

In a polymer electrolyte membrane prepared using a polymer including a monomer derived from a compound according to one embodiment of the present specification, phase separation readily occurs, and resultantly, ion conductivity is enhanced.

A polymer electrolyte membrane according to one embodiment of the present specification has excellent durability.

A fuel cell and/or a redox flow battery including the polymer electrolyte membrane have excellent durability and efficiency.

### [Description of Drawings]

FIG. 1 is a schematic diagram showing a principle of electricity generation of a fuel cell.
FIG. 2 is a diagram schematically illustrating a general structure of a redox flow battery.
FIG. 3 is a diagram schematically illustrating one embodiment of a fuel cell.

### [Reference Numeral]

- 100:: Electrolyte Membrane
- 200a:: Anode
- 200b:: Cathode
- 10, 20:: Tank
- 11, 21:: Pump
- 31:: Electrolyte Membrane
- 32:: Positive Electrode Cell
- 33:: Negative Electrode Cell
- 41:: Positive Electrode Liquid Electrolyte
- 42:: Negative Electrode Liquid Electrolyte
- 60:: Stack
- 70:: Oxidizer Supplying Unit
- 80:: Fuel Supplying Unit
- 81:: Fuel Tank
- 82:: Pump

### [Mode for Disclosure]

Hereinafter, the present specification will be described in more detail.

One embodiment of the present specification provides a compound represented by Chemical Formula 1.

Specifically, the compound represented by Chemical Formula 1 includes a sulfonyl group (-SO₂-) as a linker connecting two benzene rings. In existing technologies, a carbonyl group has been used as a linker. Using a structure introducing a sulfonyl group instead of a carbonyl group as in the compound represented by Chemical Formula 1 has an advantage of enhancing durability of an electrolyte membrane including the same. This is an effect caused by chemical stability of a sulfonyl group that is not readily decomposed by strong acids of the electrolyte or radicals generated in the electrolyte.

Meanwhile, in the compound represented by Chemical Formula 1, R1 and R2 that are each a halogen group or a hydroxyl group are each located in an ortho position and a para position with respect to the sulfonyl group (-SO₂), a linker connecting the two benzene rings. Due to an electron withdrawing effect of the sulfonyl group, reactivity of R1 and R2 located in ortho and para with respect to the sulfonyl group readily increases in a nucleophilic substitution reaction of the benzene ring, and as a result, there is an advantage in that reactivity is enhanced in the polymerization reaction.

According to one embodiment of the present specification, M, a group 1 element, may be Li, Na or K.

According to one embodiment of the present specification, R1 and R2 are each fluorine (F), and in this case, the advantage may be maximized.

According to one embodiment of the present specification, R3 is S, NH or SO₂.

According to one embodiment of the present specification, R3 is S or SO₂. In this case, there is an advantage in that the compound represented by Chemical Formula 1 is more readily synthesized.

According to one embodiment of the present specification, R3 is SO₂. When R3 is SO₂, an electrolyte membrane including the same may exhibit enhanced durability due to chemical stability of the sulfonyl group that is not readily decomposed by strong acids of the electrolyte or radicals generated in the electrolyte.

Meanwhile, as the n value increases, the length of the pendant in a polymer including a monomer derived from the compound represented by Chemical Formula 1 increases, and as a result, phase separation readily occurs due to favorable agglomeration of hydrophilic parts, which leads to an advantage of exhibiting excellent ion conductivity when used in a polymer electrolyte membrane.

According to one embodiment of the present specification, R4 is -SO₃H, -SO₃⁻M⁺, -OCF₂CF₂SO₃H or -OCF₂CF₂ SO₃⁻M⁺. R4 is included in the polymer electrolyte membrane and performs a role of an ion transfer functional group. A sulfonic acid group (-SO₃H) absorbs a maximum of approximately 10 mole of water per 1 mole, and exhibits high proton conductivity of approximately 0.1 Scm⁻¹.

According to one embodiment of the present specification, the compound represented by Chemical Formula 1 may be any one selected from among the following structures:

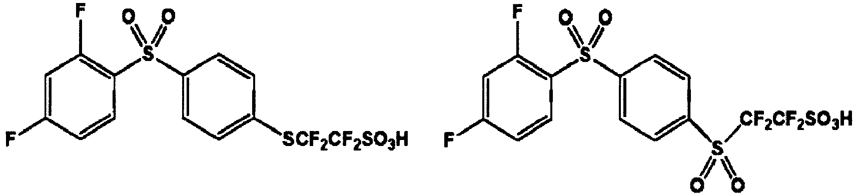

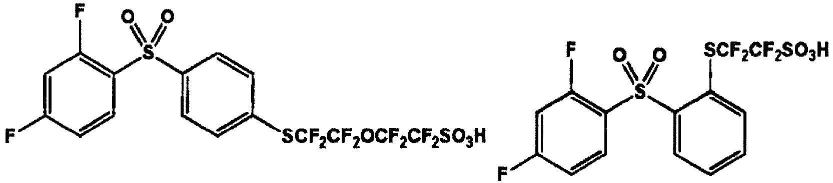

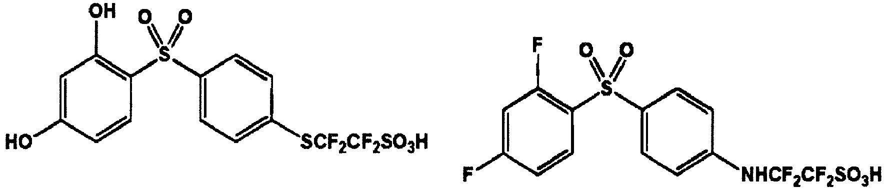

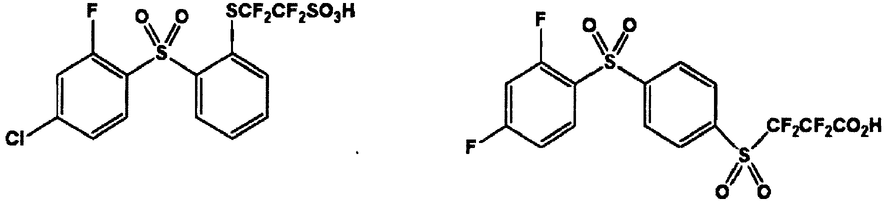

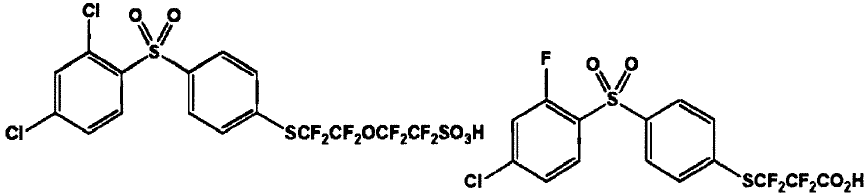

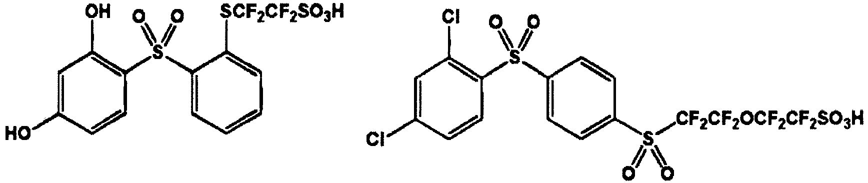

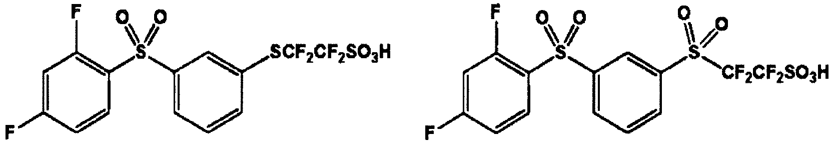

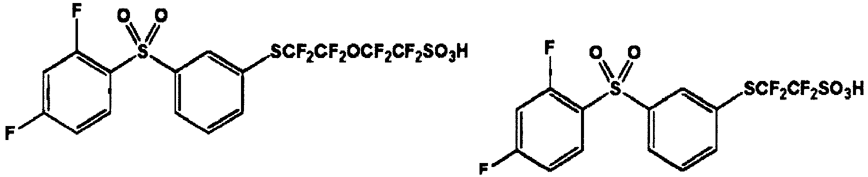

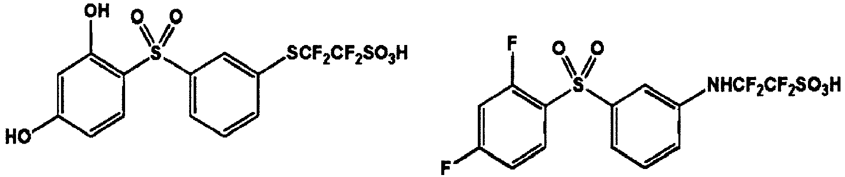

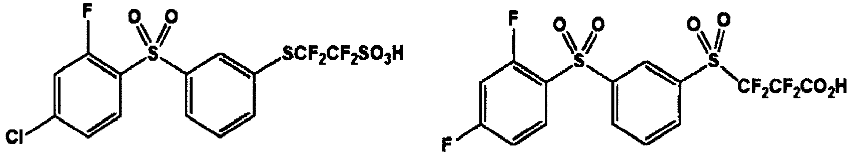

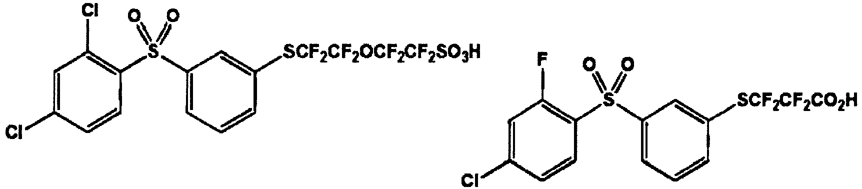

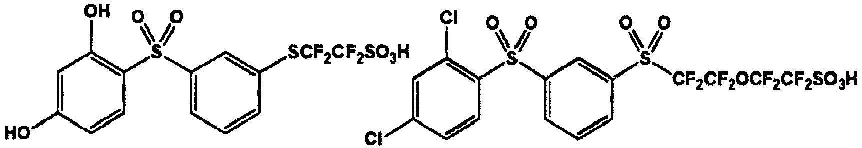

One embodiment of the present specification also provides a polymer including a monomer derived from the compound represented by Chemical Formula 1. As described above, the monomer has an advantage of exhibiting enhanced reactivity in the polymerization reaction.

In the polymer according to one embodiment of the present specification, the benzene ring substituted with R1 and R2 becomes a main chain, and with the sulfonyl group (-SO₂-) as a linker, the benzene ring substituted with [-R3(CF₂)nR4] is linked thereto in a pendant form.

In the present specification, the "monomer" means a structure including a compound in a divalent or higher form in a polymer by a polymerization reaction. Specifically, the monomer derived from the compound represented by Chemical Formula 1 may have a structure as follows. However, the structure is not limited thereto.

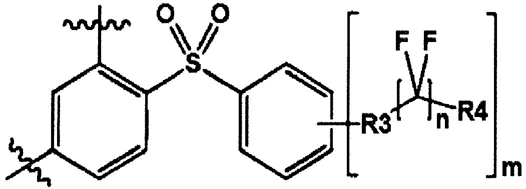

As described above, the polymer according to one embodiment of the present specification includes a monomer derived from the compound represented by Chemical Formula 1. Accordingly, an ion transfer functional group including a partial fluorine-based is present in a pendant form in the polymer, and an ion transfer functional groups are readily agglomerated in the polymer facilitating phase separation, and therefore, ion channels are readily formed, which resultantly leads to an advantage of obtaining an effect of enhancing ion conductivity in a polymer electrolyte membrane including the polymer.

According to one embodiment of the present specification, the polymer may be a random polymer. In this case, a polymer having a high molecular weight may be obtained using a simple polymerization method.

Herein, the monomer derived from the compound represented by Chemical Formula 1 performs a role of controlling ion conductivity of a polymer electrolyte membrane including the polymer, and a co-monomer (second monomer) in the remaining ratio polymerized in a random form performs a role of enhancing mechanical strength.

According to one embodiment of the present specification, the monomer derived from the compound represented by Chemical Formula 1 may be included in 0.1 mol% to 100 mol% of the whole polymer. Specifically, the polymer includes only the monomer derived from the compound represented by Chemical Formula 1. In another embodiment, the polymer may further include a second monomer in addition to the monomer derived from the compound represented by Chemical Formula 1. In this case, the content of the monomer derived from the compound represented by Chemical Formula 1 is preferably from 0.5 mol% to 65 mol%. More preferably, the content may be from 5 mol% to 65 mol%. A polymer including the monomer derived from the compound in the above-mentioned range has mechanical strength and high ion conductivity.

As the second monomer, those known in the art may be used. Herein, the second monomer may be used in one, two or more types.

Examples of the second monomer may include monomers forming perfluorosulfonic acid polymers, hydrocarbon-based polymers, polyimide, polyvinylidene fluoride, polyether sulfone, polyphenylene sulfide, polyphenylene oxide, polyphosphazene, polyethylene naphthalate, polyester, doped polybenzimidazole, polyetherketone, polysulfone, acids thereof, or bases thereof.

According to one embodiment of the present specification, the content of the co-monomer that is the second monomer in the polymer may be greater than 0% by weight and less than or equal to 99.9% by weight.

According to one embodiment of the present specification, when the polymer includes the second monomer, the polymer may be a random polymer.

One embodiment of the present specification also provides a polymer electrolyte membrane including the polymer. The polymer electrolyte membrane may exhibit effects described above.

In the present specification, the "electrolyte membrane" includes, as a membrane capable of exchanging ions, a membrane, an ion-exchange membrane, an ion-transfer membrane, an ion-conductive membrane, a separator, an ion-exchange separator, an ion-transfer separator, an ion-conductive separator, an ion-exchange electrolyte membrane, an ion-transfer electrolyte membrane, an ion-conductive electrolyte membrane or the like.

The polymer electrolyte membrane according to the present specification may be prepared using materials and/or methods known in the art except that the polymer electrolyte membrane includes a monomer derived from the compound represented by Chemical Formula 1.

According to another embodiment, a weight average molecular weight of the polymer included in the polymer electrolyte membrane may be greater than or equal to 500 and less than or equal to 5,000,000 (g/mol), specifically, greater than or equal to 20,000 and less than or equal to 2,000,000 (g/mol), and more specifically, greater than or equal to 50,000 and less than or equal to 1,000,000 (g/mol).

When the copolymer has a weight average molecular weight of greater than or equal to 500 and less than or equal to 5,000,000 (g/mol), mechanical properties of the electrolyte membrane do not decline, and proper polymer solubility is maintained, and as a result, the electrolyte membrane may be readily manufactured.

According to one embodiment of the present specification, the polymer electrolyte membrane has an ion exchange capacity (IEC) value of 0.01 mmol/g to 5 mmol/g. When the polymer electrolyte membrane has an ion exchange capacity value in the above-mentioned range, ion channels are formed in the polymer electrolyte membrane, and the polymer may exhibit ion conductivity.

According to one embodiment of the present specification, the electrolyte membrane may have a thickness of 1 µm to 500 µm, and specifically 10 µm to 200 µm. When the electrolyte membrane has a thickness of 1 µm to 500 µm, electric short and electrolyte material cross over are reduced, and an excellent cation conductivity property may be exhibited.

According to one embodiment of the present specification, the polymer electrolyte membrane has ion conductivity of greater than or equal to 0.001 S/cm and less than or equal to 0.5 S/cm, and specifically, greater than or equal to 0.01 S/cm and less than or equal to 0.5 S/cm.

According to another embodiment, ion conductivity of the polymer electrolyte membrane may be measured under a humidity condition. The humidity condition may mean a full humidity condition, may mean relative humidity (RH) of 10% to 100%, or may mean relative humidity (RH) of 30% to 100%.

According to one embodiment of the present specification, the polymer electrolyte membrane may have ion conductivity of greater than or equal to 0.001 S/cm and less than or equal to 0.5 S/cm, and the ion conductivity may be measured under relative humidity (RH) of 10% to 100%. According to another embodiment, the polymer electrolyte membrane may have ion conductivity of greater than or equal to 0.01 S/cm and less than or equal to 0.5 S/cm, and the ion conductivity may be measured under relative humidity (RH) of 30% to 100%.

According to one embodiment of the present specification, at least a part of the polymer may have a metal salt form. In addition, the metal salt may be substituted with an acid form.

Specifically, an electrolyte membrane including a polymer substituted with hydrogen (H) instead of metal M may be formed by adding an acid solution to a polymer having -SO₃⁻M⁺ or - OCF₂CF₂SO₃⁻M⁺ as R4 in Chemical Formula 1.

According to one embodiment of the present specification, general acid solutions may be used in the acid treatment, and specifically, hydrochloric acid or sulfuric acid may be used.

According to one embodiment of the present specification, the acid solution may have a concentration of greater than or equal to 0.1 M and less than or equal to 10 M, and specifically, greater than or equal to 1 M and less than or equal to 2 M. When the acid solution has a concentration of greater than or equal to 0.1 M and less than or equal to 10 M, substitution with hydrogen instead of metal M may be readily carried out without damaging the electrolyte membrane.

One embodiment of the present specification also provides a membrane-electrode assembly including an anode; a cathode; and the polymer electrolyte membrane described above provided between the anode and the cathode.

The membrane-electrode assembly (MEA) means an assembly of electrodes (cathode and anode) in which an electrochemical catalyst reaction of fuel and air occurs and a polymer membrane in which hydrogen ion transfer occurs, and is a single assembled unit in which electrodes (cathode and anode) and an electrolyte membrane are adhered.

The membrane-electrode assembly of the present specification has a form of a catalyst layer of an anode and a catalyst layer of a cathode being brought into contact with an electrolyte membrane, and may be prepared using common methods known in the art. As one example, the membrane-electrode assembly may be prepared through thermocompressing the cathode; the anode; and the electrolyte membrane located between the cathode and the anode at 100°C to 400°C while sticking these together.

The anode electrode may include an anode catalyst layer and an anode gas diffusion layer. The anode gas diffusion layer may again include an anode micropore layer and an anode electrode substrate.

The cathode electrode may include a cathode catalyst layer and a cathode gas diffusion layer. The cathode gas diffusion layer may again include a cathode micropore layer and a cathode electrode substrate.

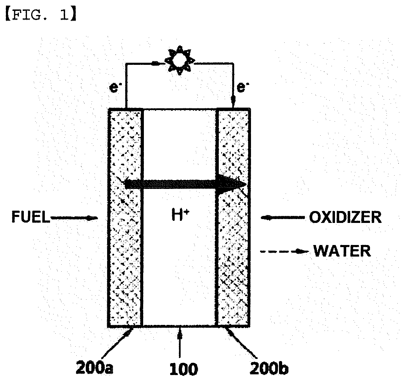

FIG. 1 is a schematic diagram showing a principle of electricity generation of a fuel cell, and in the fuel cell, a most basic unit generating electricity is a membrane-electrode assembly (MEA), and this is formed with an electrolyte membrane (100), and anode (200a) and cathode (200b) electrodes formed on both sides of the electrolyte membrane (100). When referring to FIG. 1 showing a principle of electricity generation of a fuel cell, an oxidation reaction of fuel such as hydrogen or hydrocarbon such as methanol and butane occurs in the anode (200a) to generate hydrogen ions (H⁺) and electrons (e⁻), and the hydrogen ions migrate to the cathode (200b) through the electrolyte membrane (100). In the cathode (200b), water is produced through the reaction of the hydrogen ions transferred through the electrolyte membrane (100), an oxidizer such oxygen, and electrons. Electrons migrate to an external circuit through such a reaction.

The anode electrode catalyst layer is a place where an oxidation reaction of fuel occurs, and catalysts selected from the group consisting of platinum, ruthenium, osmium, platinum-ruthenium alloys, platinum-osmium alloys, platinum-palladium alloys and platinum-transition metal alloys may be preferably used. The cathode electrode catalyst layer is a place where a reduction reaction of an oxidizer occurs, and platinum or platinum-transition metal alloys may be preferably used as catalysts. The catalysts may be used as they are, or may be used while being supported on a carbon-based carrier.

The process of introducing the catalyst layer may be carried out using common methods known in the art, and for example, a catalyst ink may be directed coated on the electrolyte membrane, or a catalyst ink may be coated on the gas diffusion layer to form the catalyst layer. Herein, the coating method of the catalyst ink is not particularly limited, and methods of spray coating, tape casting, screen printing, blade coating, die coating, spin coating or the like may be used. The catalyst ink may be typically formed with a catalyst, a polymer ionomer and a solvent.

The gas diffusion layer becomes a migration path of reaction gases and water while performing a role of a current conductor, and has a porous structure. Accordingly, the gas diffusion layer may be formed including a conductive substrate. As the conductive substrate, carbon paper, carbon cloth or carbon felt may be preferably used. The gas diffusion layer may be formed further including a micropore layer between the catalyst layer and the conductive substrate. The micropore layer may be used for enhancing fuel cell performance under a low humidity condition, and performs a role of allowing the electrolyte membrane to be under a sufficiently wet condition by having the amount of water escaping outside the gas diffusion layer being small.

One embodiment of the present specification provides a polymer electrolyte-type fuel cell including two or more of the membrane-electrode assemblies; a stack including a bipolar plate provided between the membrane-electrode assemblies; a fuel supplying unit supplying fuel to the stack; and an oxidizer supplying unit supplying an oxidizer to the stack.

When using the electrolyte membrane according to one embodiment of the present specification as an ion-exchange membrane of a fuel cell, effects described above may be obtained.

A fuel cell is an energy conversion device directly converting chemical energy of fuel into electric energy. In other words, a fuel cell employs a power generation method utilizing a fuel gas and an oxidizer, and using electrons generated during the oxidation and reduction reactions thereof to produce power.

The fuel cell may be prepared through common methods known in the art using the membrane-electrode assembly (MEA) described above. For example, the fuel cell may be prepared forming with the membrane-electrode assembly (MEA) prepared above and a bipolar plate.

The fuel cell of the present specification is formed including a stack, a fuel supplying unit and an oxidizer supplying unit.

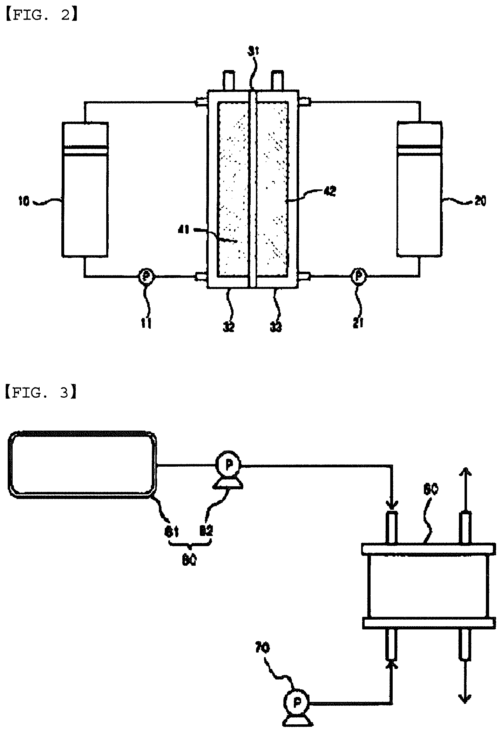

FIG. 3 is a diagram schematically illustrating the fuel cell, and the fuel cell is formed including a stack (60), an oxidizer supplying unit (70) and a fuel supplying unit (80).

The stack (60) includes one, two or more of the membrane-electrode assemblies described above, and when two or more of the membrane-electrode assemblies are included, a separator provided therebetween is included. The separator prevents the membrane-electrode assemblies from being electrically connected, and performs a role of transferring fuel and oxidizer supplied from the outside to the membrane-electrode assemblies.

The oxidizer supplying unit (70) performs a role of supplying an oxidizer to the stack (60). As the oxidizer, oxygen is typically used, and oxygen or air may be injected with a pump (70) to be used.

The fuel supplying unit (80) performs a role supplying fuel to the stack (60), and may be formed with a fuel tank (81) storing fuel, and a pump (82) supplying the fuel stored in the fuel tank (81) to the stack (60). As the fuel, hydrogen or hydrocarbon fuel in a gas or liquid state may be used. Examples of the hydrocarbon fuel may include methanol, ethanol, propanol, butanol or natural gas.

The fuel cell may include a polymer electrolyte fuel cell, a direct liquid fuel cell, a direct methanol fuel cell, a direct formic acid fuel cell, a direct ethanol fuel cell, a direct dimethyl ether fuel cell or the like.

In addition, one embodiment of the present specification provides a redox flow battery including a positive electrode cell including a positive electrode and a positive electrode liquid electrolyte; a negative electrode cell including a negative electrode and a negative electrode liquid electrolyte; and the polymer electrolyte membrane according to one embodiment of the present specification provided between the positive electrode cell and the negative electrode cell.

A redox flow battery (oxidation-reduction flow battery) is a system charged and discharged by active materials included in a liquid electrolyte being oxidized and reduced, and is an electrochemical storage device directly storing chemical energy of the active materials as electric energy. A redox flow battery uses a principle of being charged and discharged from the exchange of electrons occurring when liquid electrolytes including active materials in different oxidation states meet with an ion-exchange membrane in between. A redox flow battery is generally formed with a tank holding a liquid electrolyte, a battery cell where charge and discharge occur, and a circulating pump for circulating the liquid electrolyte between the tank and the battery cell, and a unit cell of the battery cell includes an electrode, an electrolyte and an ion-exchange membrane.

When using the electrolyte membrane according to one embodiment of the present specification as an ion-exchange membrane of the redox flow battery, effects described above may be obtained.

The redox flow battery of the present specification may be prepared using common methods known in the art except that the redox flow battery includes the polymer electrolyte membrane according to one embodiment of the present specification.

As illustrated in FIG. 2, the redox flow battery is divided into a positive electrode cell (32) and a negative electrode cell (33) by an electrolyte membrane (31). The positive electrode cell (32) and the negative electrode cell (33) include a positive electrode and a negative electrode, respectively. The positive electrode cell (32) is connected to a positive electrode tank (10) for supplying and releasing a positive electrode liquid electrolyte (41) through a pipe. The negative electrode cell (33) is also connected to a negative electrode tank (20) for supplying and releasing a negative electrode liquid electrolyte (42) through a pipe. The liquid electrolytes circulate through pumps (11, 21), and through an oxidation/reduction reaction (that is, a redox reaction) changing the oxidation number of ions, charge and discharge occur in the positive electrode and the negative electrode.

One embodiment of the present specification also provides a method for preparing the polymer electrolyte membrane. The polymer electrolyte membrane may be prepared using materials and/or methods known in the art except that a polymer including a monomer derived from the compound represented by Chemical Formula 1 is included. For example, the polymer electrolyte membrane may be prepared by preparing a polymer solution through adding the polymer to a solvent, and then forming a film using a solution casting method.

## Claims

1. A compound represented by the following Chemical Formula 1: 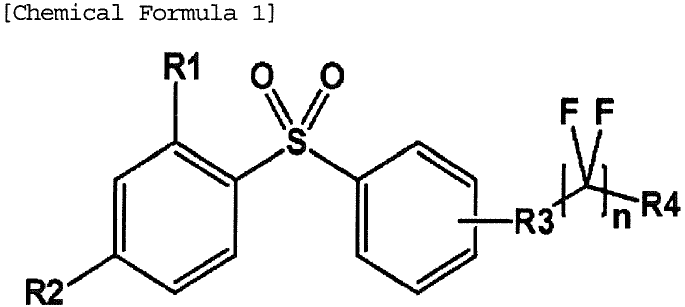
wherein, in Chemical Formula 1,
R1 and R2 are the same as or different from each other, and each independently a halogen group or a hydroxyl group;
R3 is S, O, CF₂, NH or SO₂;
R4 is -SO₃H, -SO₃⁻M⁺, -COOH, -COO⁻M⁺, -OCF₂CF₂SO₃H or - OCF₂CF₂SO₃⁻M⁺;
M is a group 1 element; and
n is an integer of 2 to 20.

2. The compound of Claim 1, wherein R3 is S, NH or SO₂.

3. The compound of Claim 1, wherein R3 is S or SO₂.

4. The compound of Claim 1, wherein R1 and R2 are fluorine (F) .

5. The compound of Claim 1, wherein R4 is -SO₃H, -SO₃⁻M⁺, - OCF₂CF₂SO₃H or -OCF₂CF₂SO₃⁻M⁺.

6. The compound of Claim 1, wherein the compound represented by Chemical Formula 1 is any one selected from among the following structures:

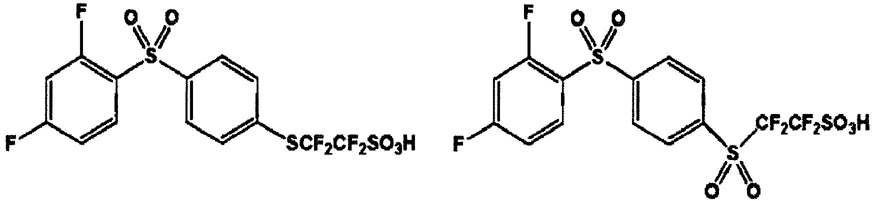

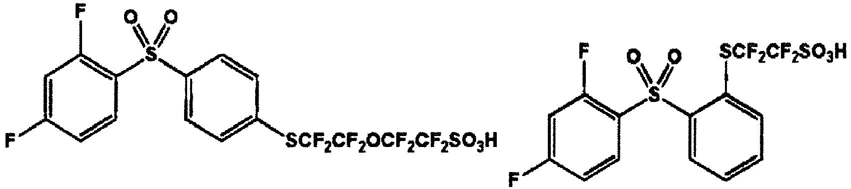

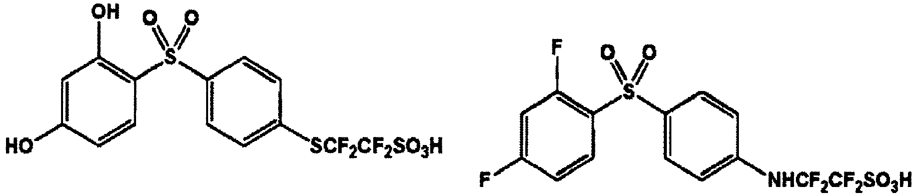

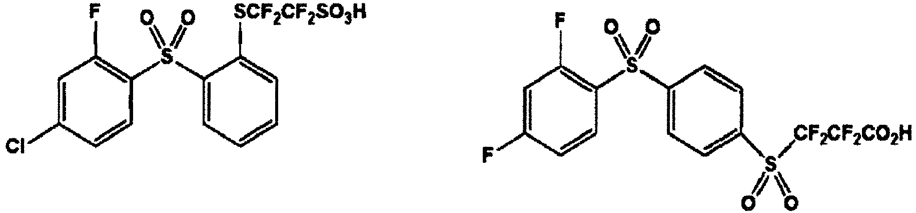

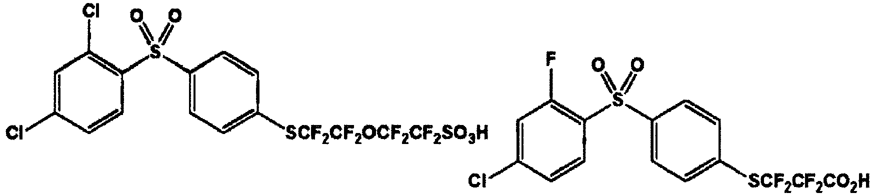

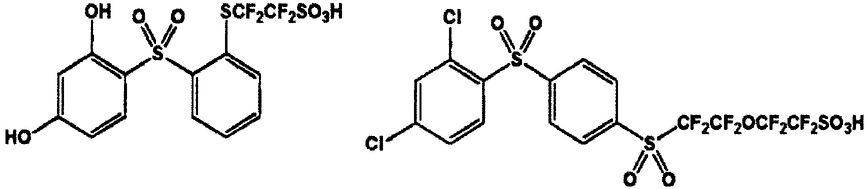

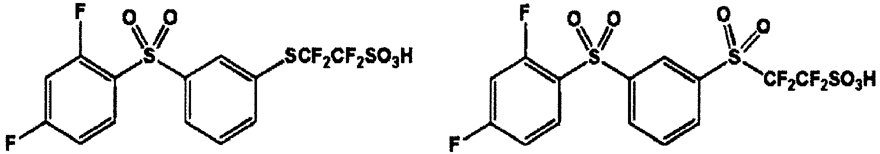

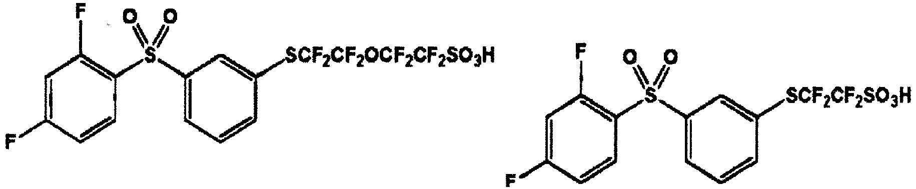

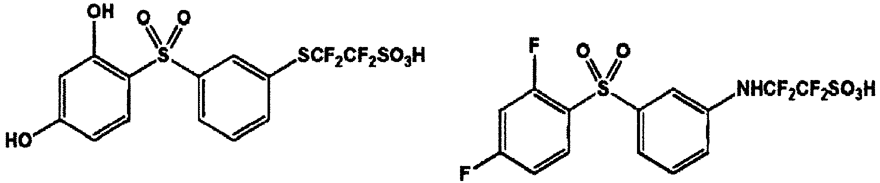

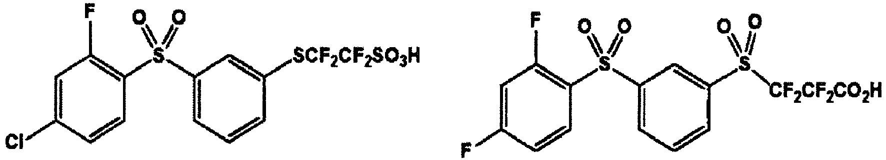

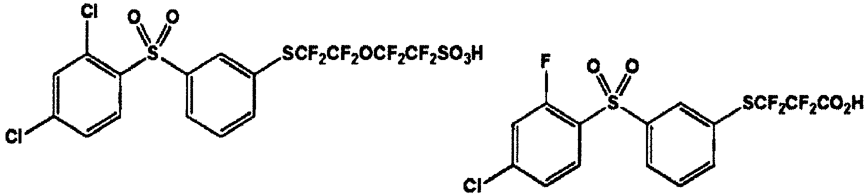

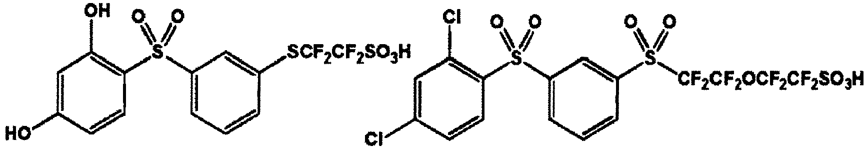

7. A polymer comprising a monomer derived from the compound of any one of Claims 1 to 6.

8. A polymer electrolyte membrane comprising the polymer of Claim 7.

9. The polymer electrolyte membrane of Claim 8, wherein the polymer has a weight average molecular weight of greater than or equal to 500 and less than or equal to 5,000,000 (g/mol).

10. The polymer electrolyte membrane of Claim 8, which has a thickness of greater than or equal to 1 µm and less than or equal to 500 µm.

11. The polymer electrolyte membrane of Claim 8, which has ion conductivity of greater than or equal to 0.01 S/cm and less than or equal to 0.5 S/cm.

12. The polymer electrolyte membrane of Claim 8, which has an ion exchange capacity (IEC) value of 0.01 mmol/g to 5 mmol/g.

13. A membrane-electrode assembly comprising:
an anode;
a cathode; and
the polymer electrolyte membrane of Claim 8 provided between the anode and the cathode.

14. A polymer electrolyte-type fuel cell comprising:
two or more of the membrane-electrode assemblies of Claim 13;
a stack including a bipolar plate provided between the membrane-electrode assemblies;
a fuel supplying unit supplying fuel to the stack; and
an oxidizer supplying unit supplying an oxidizer to the stack.

15. A redox flow battery comprising:
a positive electrode cell including a positive electrode and a positive electrode liquid electrolyte;
a negative electrode cell including a negative electrode and a negative electrode liquid electrolyte; and
the polymer electrolyte membrane of Claim 8 provided between the positive electrode cell and the negative electrode cell.
